(19) Europäisches Patentamt
European Patent Office
Office européen des brevets

(11) Veröffentlichungsnummer: **0 261 388 B1**

(12) # EUROPÄISCHE PATENTSCHRIFT

(45) Veröffentlichungstag der Patentschrift: **04.04.90**

(51) Int. Cl.⁴: **C07C 69/743**, C07C 255/03, C07C 61/40, C07C 67/48, C07C 51/487, A01N 53/00

(21) Anmeldenummer: **87111922.8**

(22) Anmeldetag: **18.08.87**

(54) **Verfahren zur Diastereomerentrennung von Cyclopropancarbonsäureestern.**

(30) Priorität: **29.08.86 DE 3629387**

(43) Veröffentlichungstag der Anmeldung: **30.03.88 Patentblatt 88/13**

(45) Bekanntmachung des Hinweises auf die Patenterteilung: **04.04.90 Patentblatt 90/14**

(84) Benannte Vertragsstaaten: **BE CH DE FR GB IT LI NL**

(56) Entgegenhaltungen: **EP-A- 0 046 950**

(73) Patentinhaber: **BAYER AG, D-5090 Leverkusen 1 Bayerwerk(DE)**

(72) Erfinder: **Fuchs, Rainer, Dr., Röberstrasse 8, D-5600 Wuppertal 1(DE)**
Erfinder: **Stendel, Wilhelm, Dr., In den Birken 55, D-5600 Wuppertal 1(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 261 388 B1

## Beschreibung

Verfahren zur Diastereomerentrennung von Cyclopropancarbonsäureestern

Die vorliegende Erfindung betrifft ein Verfahren zur Trennung von zueinander diastereomeren Formen von Cyclopropancarbonsäureestern, dafür verwendbare neue Verbindungen sowie deren Herstellung sowie ein Verfahren zur Reinigung von technisch hergestellten Cyclopropancarbonsäureestern und der ihnen zugrundeliegenden Säuren, sowie ein neues Enantiomerenpaar von Flumethrin.

Cyclopropancarbonsäuren sowie die aus ihnen hergestellten Wirkstoffe der Klasse der Pyrethroide fallen bei ihrer Herstellung als Gemische ihrer optischen und sterischen Isomeren an. Diese Isomeren der Pyrethroide besitzen unterschiedliche Wirksamkeiten gegenüber Schädlingen gegen die sie eingesetzt werden. Es ist daher von Interesse z.B. die sterischen Isomeren zu trennen.

Pyrethroide sind gegenüber üblichen physikalischen Reinigungsmethoden wie z.B. Destillation empfindlich. Ihre Umkristallisation scheitert oft daran, daß sie als Öle vorliegen, sowie an ihrem ungünstigen Lösungsverhalten. Andererseits enthalten die technisch hergestellten Pyrethroide oftmals Verunreinigungen die vor dem Einsatz der Wirkstoffe entfernt werden müssen. Diese Reinigung ist im technischen Maßstab jedoch nur mit erheblichem Aufwand möglich. Es war daher von Interesse ein auch in technischem Maßstab einfach und preiswert durchzuführendes Reinigungsvrfahren für Pyrethroide zur Verfügung zu haben.

Pyrethroide der folgenden Formel

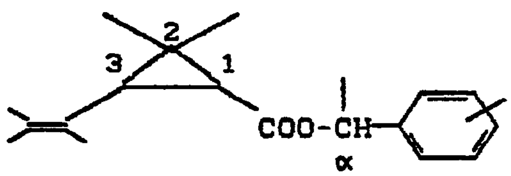

besitzen an den C-Atomen 1, 3 und α Asymmetriezentren. Sie liegen daher in Form folgender Isomeren und ihrer Gemische vor:

| | | | | | | | |
|---|---|---|---|---|---|---|---|
| a) | 1S | 3R | αS | e) | 1S | 3S | αS |
| b) | 1R | 3S | αR | f) | 1R | 3R | αR |
| c) | 1S | 3R | αR | g) | 1S | 3S | αR |
| d) | 1R | 3S | αS | h) | 1R | 3R | αS |

Die Isomeren a) und b), c) und d), e) und f) sowie g) und h) sind enantiomer zueinander. Alle Isomeren die nicht enantiomer zueinander sind, sind diastereomer zueinander.

Die zueinander enantiomeren Formen sind spiegelbildlich zueinander und unterscheiden sich nur in asymmetrischer Umgebung z.B. durch den Drehwert des polarisierten Lichtes ihrer Lösungen. Sie besitzen keine unterschiedlichen chemischen und physikalischen Eigenschaften durch die sie sich trennen lassen. Eine Trennung läßt sich nur mit einem chiralen Hilfsreagenz bewerkstelligen.

Die zueinander diastereomeren Formen unterscheiden sich durch den relativen Abstand der Atome im Molekül zueinander. Sie besitzen daher unterschiedliche physikalische und chemische Eigenschaften.

Die einzelnen Isomeren besitzen unterschiedliche biologische Eigenschaften. Es ist daher von Interesse die wirksamen von den weniger wirksamen Isomeren zu trennen. Da sich eine Trennung der zueinander enantiomeren Isomeren nur mit teuren chiralen Hilfsreagenzien durchführen läßt, ist es sinnvoll die zueinander diastereomeren Isomeren zu trennen.

Grundsätzlich ist dieses Trennung durch Destillation, Umkristallisation oder Chromatographie möglich. Im Falle der zum Teil thermisch empfindlichn Pyrethroide ist eine Destillation zumindest im technischen Maßstab nicht möglich. Auch eine Trennung über Chromatographie ist im technischen Maßstab zu aufwendig und kostspielig. Die Umkristallisation scheitert im technischen Maßstab vielfach daran, daß die Verbindungen ölig anfallen oder daß es schwierig ist geeignete Lösungsmittelsysteme, in denen sie durchgeführt werden kann, zu finden.

Es wurde ein Verfahren zur Trennung von zueinander diastereomeren Formen der Cyclopropancarbonsäureester der Formel (I) gefunden

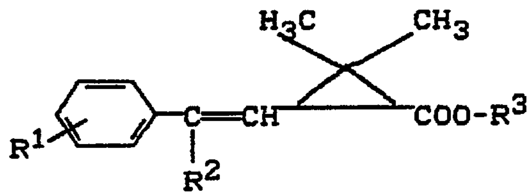

(I)

in welcher
$R^1$ für Wasserstoff oder Halogen
$R^2$ für Wasserstoff oder Halogen
$R^3$ für einen Rest der Formel a)

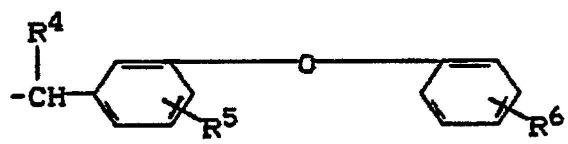

(a)

wobei
$R^4$ für Wasserstoff, CN, $-C{\equiv}CH$ oder $CH_3$,
$R^5$ für Wasserstoff oder Halogen,
$R^6$ für Wasserstoff oder Halogen steht,
das dadurch gekennzeichnet ist, daß man Verbindungen der Formel (I) unter Erwärmen in Gegenwart eines $C_{1-4}$-Alkanols in einer ersten Stufe mit Verbindungen der Formel (II)

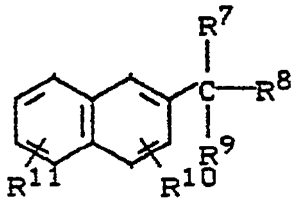

(II)

in welcher
$R^7$, $R^8$, $R^9$ für Wasserstoff oder Methyl stehen oder gemeinsam mit dem C-Atom an das sie gebunden sind sowie den diesem benachbarten C-Atomen des Grundkörpers einen ankondensierten 6-Ring bilden,
$R^{10}$ für Wasserstoff, Methyl, Ethyl, Halogen, Amino, Methylamino, Dimethylamino steht,
$R^{11}$ für Wasserstoff, Methyl oder Ethyl steht

zu einem Komplex im Verhältnis I:II wie 1:1 oder 2:1 umsetzt, und in einer zweiten Stufe die schwerer löslichen Komplexe eines Teils der Diastereomeren auskristallisieren läßt und abtrennt und gegebenenfalls in einer dritten Stufe die abgetrennten Komplexe in Gegenwart eines Lösungsmittels und in Gegenwart einer anorganischen oder organischen Base epimerisiert und erneut der ersten Stufe des Verfahrens zuführt.

Es wurde ferner ein Verfahren zur Reinigung von Cyclopropancarbonsäureester der Formel (I) und der ihnen zugrundeliegenden Säuren von chemischen Verunreinigungen gefunden, das dadurch gekennzeichnet ist, daß man die Verbindungen der Formel (I) in Gegenwart von $C_{1-4}$-Alkanolen und in Gegenwart von anorganischen oder organischen Basen mit Verbindungen der Formel (II) im Verhältnis von etwa 1:1 oder 2:1 umsetzt und die dabei entstehenden schwerlöslichen Komplexe abtrennt.

Gegenstand der vorliegenden Erfindung sind außerdem Komplexe der Formel (III)

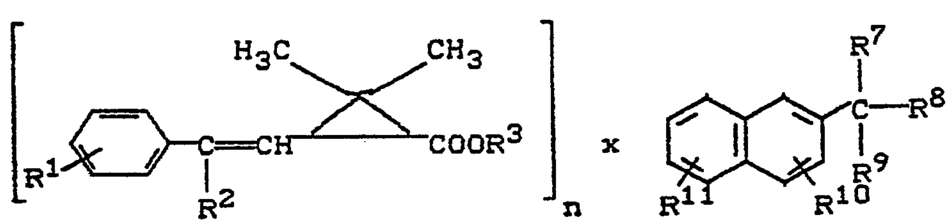

(III)

in welcher
$R^1$,$R^2$,$R^3$,$R^7$ bis $R^{11}$ die oben angegebenen Bedeutung haben
n für 1 oder 2 steht.

Bevorzugt werden Verbindungen der Formel (I) eingesetzt, in der
$R^1$ für Wasserstoff oder Chlor steht,
$R^2$ für Chlor steht,

$R^3$ für den Rest der Formel a) steht, in welcher
$R^4$ für Wasserstoff oder CN steht,
$R^5$ für Wasserstoff oder Fluor steht,
$R^6$ für Wasserstoff steht.

Besonders bevorzugt wird Flumethrin der Formel

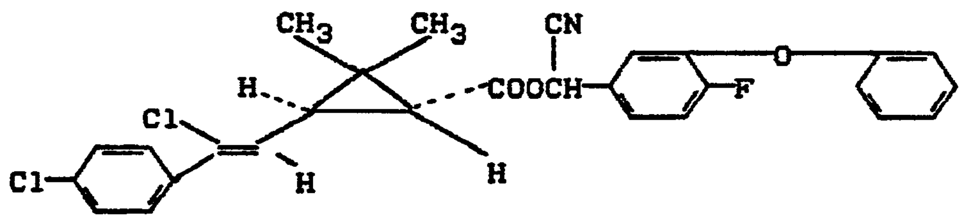

eingesetzt. Flumethrin liegt dabei besonders bevorzugt als Gemisch seiner trans Z Isomeren vor (Z bezieht sich auf die Stellung der Substituenten an der Vinyl-Doppelbindung; trans bezieht sich auf die Stellung der Substituenten an den C-Atomen C1 und C3 des Cyclopropancarbonsäureringes),

Bevorzugt werden Verbindungen der Formel (II) eingesetzt, in welcher $R^7$, $R^8$, $R^9$ für Wasserstoff stehen oder gemeinsam mit dem C-Atom an das sie gebunden sind sowie den diesen benachbarten C-Atomen einen ankondensierten Benzolring bilden und
$R^{10}$ für Wasserstoff oder Methyl steht,
$R^{11}$ für Wasserstoff oder Methyl steht.
Insbesondere sei genannt:
2-Methylnaphthalin, 2,6-Dimethylnaphthalin, 2,3-Dimethylnaphthalin, Anthracen, Phenanthren.

Bei den in Frage kommenden $C_1$–$C_4$ Alkoholen handelt es sich im einzelnen um: Methanol, Ethanol, Propanole, Butanole; insbesonders genannt sei Isopropanol.

Die Komplexbildung kann auf einfache Weise durch Umsetzung von 1 Mol Verbindungen der Formel (I) mit 1 oder 0,5 Mol Verbindungen der Formel (II) in der Wärme (50°C), in Gegenwart eines der vorstehend genannten aliphatischen Alkohole erfolgen.

Technisch hergestellten Flumethrin besteht aus einem Gemisch der trans Z Isomeren 1R 3S αR and 1S 3R αS und 1R 3S αS und 1S 3R αR.

Bei technisch hergestellten Flumethrin liegen die Enantiomerenpaare z.B. in folgenden Verhältnis vor: 1R 3S αR und 1S 3R αS = 60 %, 1R 3S αS und 1S 3R αR = 40%.

Das Verfahren läßt sich auch zur technischen Reinigung z.B. von technisch hergestelltem Flumethrin (trans Z Isomere) verwenden. Flumethrin läßt sich in größerem Maßstab nur sehr schwer durch Destillation oder Umkristallisation reinigen. Durch das erfindungsgemäße Verfahren kann das technisch hergestellte chemische Verunreinigungen enthaltende Flumethrin auf einfache Weise gereinigt werden. Technisches Flumethrin (trans Z Isomere) wird in Isopropanol gelöst, mit Verbindungen der Formel (IV) in Gegenwart von anorganischen oder organischen Basen versetzt. Auf diese Weise entstehen schwerlösliche Komplexe der Isomeren 1R 3S αR und 1S 3R αS. Diese werden abgetrennt und können entweder als solche verwendet werden oder nach Auflösen in einem geeigneten Lösungsmittel in Gegenwart anorganischer oder organischer Basen zu den Isomeren 1R 3S αR, 1S 3R αS, 1R 3S αS, 1S 3R αR epimerisiert werden. Anschließend kann dieses Gemisch isoliert oder gegebenenfalls erneut der oben beschriebenen Trennung zugeführt werden.

Als Basen sind geeignet Alkali und Erdalkalihydroxide, -carbonate und -bicarbonate, Ammoniak, primäre, sekundäre und tertiäre Amine, wie Methylamin, Dimethylamin, Isopropylamin, Diisopropylamin, Triethylamin, Trimethylamin, Pyridin, Di-s-butylamin.

Besonders zu erwähnen sind die bei dem neuen Verfahren entstehenden Komplexe der Formel (III). Im Falle von Flumethrin seien dabei bevorzugt folgende Komplexe genannt:
(Flumethrin trans Z 1R-3S-αR und 1S-3R-αS)$_2$ x 2,6-Dimethylnaphthalin und
(Flumethrin trans Z 1R-3S-αR und 1S-3R-αS)$_2$ x Anthracen
(Flumethrin trans Z 1R-3S-αR und 1S-3R-αS)$_2$ x Phenanthren
(Flumethrin trans Z 1R-3S-αR und 1S-3R-αS)$_2$ x 2,3-Dimethylnaphthalin
(Flumethrn trans Z 1R-3S-αR und 1S-3R-αS)$_2$ x Methylnaphthalin.

Ihre Herstellung erfolgt in dem oben beschriebenen Verfahren. Sie lassen sich leicht durch Lösen in einem organischen Lösungsmittel wie z.B. Toluol, Chloroform spalten. Sie können aber auch als solche in insektiziden und ektoparasitiziden Mitteln bzw. für die Herstellung solcher Mittel verwendet werden.

Mit Hilfe der beschriebenen trans Z 1R 3S αR und 1S 3R αS Komplexe von Flumethrin können die weniger wirksamen Isomeren aus dem Isomerengemisch der technisch herge stellten Flumethrin abgetrennt werden. Man erhält so im Rückstand das trans Z Enantiomerenpaar 1R 3S αS und 1S 3R αR im wesentlichen frei vom Enantiomerenpaar trans Z 1R 3S αR und 1S 3S αS (< 20 %).

Das Enantiomerenpaar trans Z 1R 3S αS uns 1S 3R αR ist neu und eignet sich besonders zur Bekämpfung von Ektoparassiten.

Beispiel A

Test mit Stomoxys calcitrans

Lösungsmittel: 35 Gewichtsteile Ethylenglykolmonomethylether
35 Gewichtsteile Nonylphenolpolyglykolether.

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Stomoxys calcitrans werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigt das Gemisch aus Beispiel 3 bei einer Konzentration von 100 ppm 100 % Wirkung.

Beispiel B

Test mit Musca autumnalis

Lösungsmittel: 35 Gewichtsteile Ethylenglykolmonomethylether;35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Musca autumnalis werden in Petrischalen gebracht, die Filterpapierscheiben entsprechender Größe enthalten, die einen Tag vor Versuchsbeginn mit 1 ml der zu testenden Wirkstoffzubereitung durchtränkt wurden. Nach 3 Stunden wird der Abtötungsgrad bestimmt.

Bei diesem Test zeigt das Gemisch aus Beispiel 2 bei einer Konzentration von 100 ppm 100 % Wirkung.

Beispiel C

Test mit Lucilia cuprina res.-Larven

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

Etwa 30 Lucilia cuprina, res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigt das Gemisch aus Beispiel 3 bei einer Konzentration von 100 ppm 100 % Wirkung.

Beispiel D

Test mit Boophilus microplus resistent

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittelgemisches und verdünnt das so erhaltene Konzentrat mit Wasser auf die jeweils gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigt das Gemisch aus Beispiel 3 bei eine Konzentration von 100 ppm 100 % Wirkung.

Beispiel 1

0,51 g (1 mMol) des Isomerenpaares Z-trans 1R 3S αR und Z-trans 1S 3R αS von Flumethrin und 0,078 g (0,5 mMol) 2,6-Dimethylnaphthalin werden unter Erwärmen auf 50°C in 6 ml iso-Propanl gelöst. Nach dem Abkühlen auf Raumtemperatur kristallisiert ein Komplex aus 2 Teilen des Isomerenpaares und 1 Teil 2,6-Dimethylnaphthalin aus. Nach dem Absaugen und Trocknen erhält man 0,55 g als farblose Nadeln mit

einem Schmelzpunkt von 108 bis 109°C.

Analog werden mit dem Isomerenpaar folgende Komplexe erhalten:

| Komplex aus: A | B | Molverhältnis A : B | Schmp. |
|---|---|---|---|
| 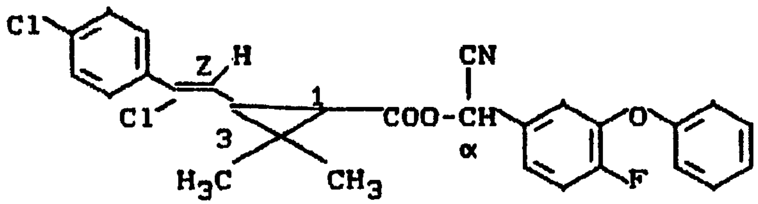 trans-Z-1R-3S-αR trans-Z-1S-3R-αS | 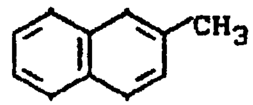 | 2 : 1 | 74° C |
| " | 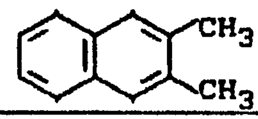 | 2 : 1 | 90° C |
| " | 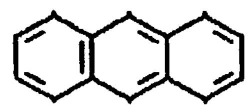 | 2 : 1 | 124° C (Zersetzung) |
| " | 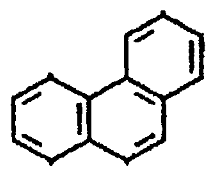 | 2 : 1 | 78° C |

Beispiel 2

102 g (0,2 Mol) technisches Flumethrin und 9,42 g (0,06 Mol) 2,6-Dimethylnaphthalin werden unter Erwärmen auf 50°C in 1000 ml iso-Propanol gelöst. Anschließend läßt man langsam unter Rühren auf Raumtemperatur abkühlen. Dabei setzt langsam Kristallisation ein, die durch Zugabe einiger der in Beispiel 1 beschriebenen Kristalle beschleunigt werden kann. Zur vollständigen Kristallisation wird die Mischung noch 24 Stunden bei Raumtemperatur gerührt. Anschließend werden die Kristalle abgesaugt, mit etwas iso-Propanol nachgewaschen und an der Luft getrocknet. Man erhält 67 g eines kristallinen Komplexes mit einem Schmelzpunkt von 92 bis 98°C und folgender Zusammensetzung:
2 Teile Isomerenpaar I
Z-trans 1R 3S αR und
Z-trans 1S 3R αS + 1 Teil 2,6-Dimethylnaphthalin (= 80 %)
2 Teile Isomerenpaar II
Z-trans 1R 3S αS und
Z-trans 1S 3R αR + 1 Teil 2,6-Dimethylnaphthalin ( = 20 %)

Beispiel 3

Das Filtrat aus Beispiel 2 wird im Vakuum vom Lösungsmittel befreit. Der Rückstand wird in Toluol gelöst und mit 30 g Kieselgel verrührt. Anschließend wird das Kieselgel abfiltriert und das Filtrat im Vakuum von Lösungsmittel befreit. Man erhält 44 g eines zähen Öls mit folgender Isomerenverteilung:
Isomerenpaar I
Z-trans 1R 3S αR und Z-trans 1S 3R αS = 16 %
Isomerenpaar II
Z-trans 1R 3S αS und Z-trans 1S 3R αR = 84 %

Beispiel 4

67 g des in Beispiel 2 erhaltenen kristallinen Komplexes mit einer Isomerenverteilung von Isomerenpaar I = 80 % und Isomerenpaar II = 20 % werden unter Erwärmen auf 70°C in 900 ml iso-Propanol gelöst und dann mit 10 g Triethylamin versetzt. Die Mischung wird 12 Stunden auf 70 bis 75°C erhitzt. Dabei ist es wichtig, eine klare Lösung zu haben. Anschließend wird das Lösungsmittel im Vakuum abdestilliert, der Rückstand in 150 ml Toluol aufgenommen und mit 100 ml verdünnter wäßriger Salzsäure ausgeschüttelt. Die organische Phase wird abgetrennt, getrocknet und das Toluol im Vakuum abdestilliert. Das verbleibende Öl = 66 g, hat ein Isomerenverhältnis von Isomerenpaar I = 65 %, Isomerenpaar II = 45 %. In einem weiteren Ansatz wurde Triethylamin entfernt, indem man es zusammen mit etwa 1/3 der iso-Propanolmenge abdestilliert. In einem weiteren Ansatz wurde Triethylamin durch Einblasen von HCl-Gas entfernt.

Beispiel 5

51 g (0,1 Mol) technisches Flumethrin (~ 86 %ig) werden unter Erwärmen auf 50°C in 500 ml iso-Propanol gelöst und mit 16 g (0,1 Mol) 2,6-Dimethylnaphthalin und 25 ml Triethylamin versetzt. Anschließend wird unter Rühren auf Raumtemperatur abgekühlt, wobei langsam Kristallisation einsetzt. Die Kristallisation kann durch Zugabe einiger Kristalle aus Beispiel 1 beschleunigt werden. Man läßt die Mischung 48 Stunden bei Raumtemperatur rühren. Anschließend werden die Kristalle abgesaugt und an der Luft getrocknet. Man erhält 50 g eines kristallinen Komplexes mit einem Schmelzpunkt von 88 bis 92°C und folgender Zusammensetzung:
2 Teile Isomerenpaar I + 1 Teil 2,6-Dimethylnaphthalin (= 80 %)
2 Teile Isomerenpaar II + 1 Teil 2,6-Dimethylnaphthalin (= 20 %)

Der kristalline Komplex wurde dann wie in Beispiel 4 beschrieben, mit Triethylamin epimerisiert. Man erhält so wieder eine Substanz mit einem Isomerenverhältnis von
Isomerenpaar I = ~ 55 %
Isomerenpaar II = ~ 45 %
und einem Gehalt von Isomerenpaar I und Isomerenpar II = 94 %.

Beispiel 6

2,85 g (0,01 Mol) (±) trans-Z-3[2-chlor-2-(4-chlorphenyl)vinyl]-2,2-dimethylcyclopropan-1-carbonsäure und 1,56 g (0,01 Mol) 2,3-Dimethylnaphthalin werden unter Erwärmen auf 50°C in 50 ml iso-Propanol gelöst. Anschließend kühlt man auf Raumtemperatur ab und rührt noch 10 Stunden bei Raumtemperatur. Die ausgefallenen Kristalle werden abgesaugt. Man erhält 1,4 g eines Komplexes aus 2 Teilen (±)-trans-Z-3-[2-chlor-2-(4-chlorphenyl)vinyl]-2,2-dimethyl-cyclopropancarbonsäure und 1 Teil 2,3-Dimethylnaphthalin als farblose Kristalle mit einem Schmelzpunkt von 128 bis 132°C.

Beispiel 7

Analog Beispiel 6 erhält man folgenden Komplex:

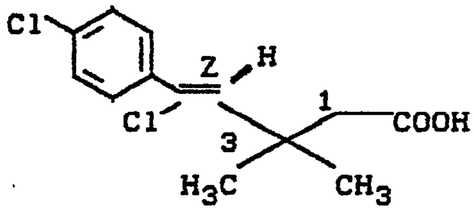

+

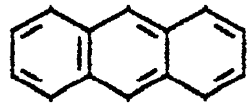

2 Teile { Z-trans-1R-3S, Z-trans-1S-3R } + 1 Teil

Schmelzpunkt des Komplexes 160°C (Zersetzung)

**Patentansprüche**

1. Verfahren zur Trennung von zueinander diastereomeren Formen der Cyclopropancarbonsäureester der Formel (I)

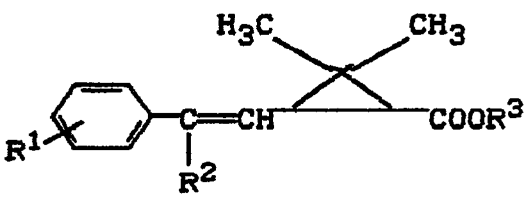

(I)

in welcher
$R^1$ für Wasserstoff oder Halogen
$R^2$ für Wasserstoff oder Halogen
$R^3$ für einen Rest der Formel a)

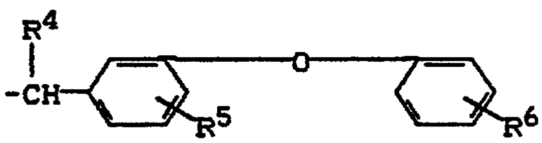

(a)

wobei
$R^4$ für Wasserstoff, CN, C≡CH oder $CH_3$,
$R^5$ für Wasserstoff oder Halogen,
$R^6$ für Wasserstoff oder Halogen steht,
das dadurch gekennzeichnet ist, daß man Verbindungen der Formel (I) unter Erwärmung in Gegenwart eines $C_{1-4}$-Alkanols in einer ersten Stufe mit Verbindungen der Formel (II)

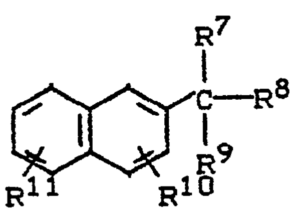

(II)

in welcher
$R^7$, $R^8$, $R^9$ für Wasserstoff oder Methyl stehen oder gemeinsam mit dem C-Atom an das sie gebunden sind sowie den diesem benachbarten C-Atomen des Grundkörpers einen ankondensierten 6-Ring bilden,

$R^{10}$ für Wasserstoff, Methyl, Ethyl, Halogen, Amino, Methylamino, Dimethylamino steht,
$R^{11}$ für Wasserstoff oder Methyl, Ethyl steht,
zu einem Komplex im Verhältnis I:II wie 1:1 oder 2:1 umsetzt, und in einer zweiten Stufe die schwer löslichen Komplexe eines Teils der Diastereomeren auskristallilieren läßt und abtrennt, und gegebenenfalls in einer dritten Stufe die abgetrennten Komplexe in Gegenwart eines Lösungsmitels und in Gegenwart einer anorganischen oder organischen Base empimerisiert und erneut der ersten Stufe des Verfahrens zuführt.

2. Verfahren zur Reinigung von Cyclopropancarbonsäureestern der Formel (I) und der ihnen zugrundeliegenden Säuren

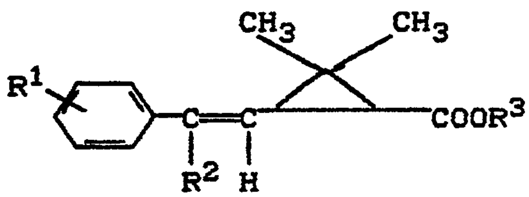

(I)

in welcher
$R^1$ für Wasserstoff oder Halogen
$R^2$ für Wasserstoff oder Halogen
$R^3$ für Wasserstoff
oder
$R^3$ für einen Rest der Formel a)

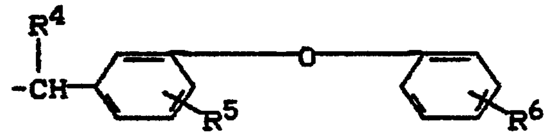

(a)

wobei
$R^4$ für Wasserstoff, CN, C≡CH oder $CH_3$,
$R^5$ für Wasserstoff oder Halogen,
$R^6$ für Wasserstoff oder Halogen steht,
von chemischen Verunreinigungen, das dadurch gekennzeichnet ist, daß man die Verbindungen der Formel (I) in Gegenwart von $C_{1-4}$-Alkanolen und in Gegenwart von anorganische oder organischen Basen mit Verbindungen der Formel (II)

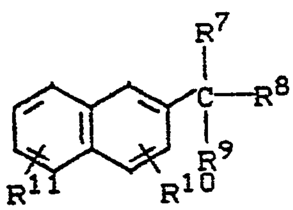

(II)

in welcher
$R^7$, $R^8$, $R^9$ für Wasserstoff oder Methyl stehen oder gemeinsam mit dem C-Atom an das sie gebunden sind sowie den diesem benachbarten C-Atomen des Grundkörpers einen ankondensierten 6-Ring bilden,
$R^{10}$ für Wasserstoff, Methyl, Ethyl, Halogen, Amino, Methylamino, Dimethylamino steht,
$R^{11}$ für Wasserstoff oder Methyl, Ethyl steht,
im Verhältnis von etwa 1:1 oder 2:1 umsetzt und die dabei entstehenden schwerlöslichen Komplexe abtrennt.

3. Komplexe der Formel (III)

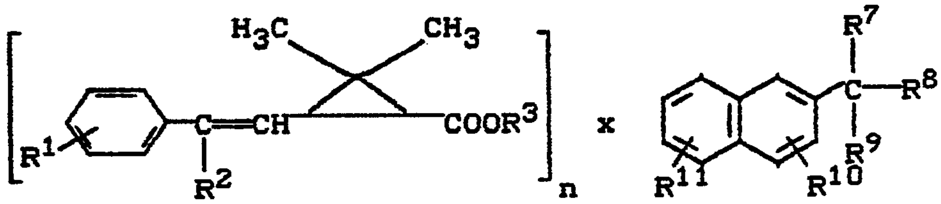

in welcher

$R^1$ für Wasserstoff oder Halogen
$R^2$ für Wasserstoff oder Halogen
$R^3$ für Wasserstoff
oder
$R^3$ für einen Rest der Formel a)

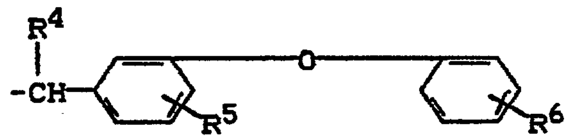

(a)

wobei
$R^4$ für Wasserstoff, CN, $-C{\equiv}CH$ oder $CH_3$,
$R^5$ für Wasserstoff oder Halogen,
$R^6$ für Wasserstoff oder Halogen steht,
$R^7$,$R^8$,$R^9$ für Wasserstoff oder Methyl stehen oder gemeinsam mit dem C-Atom an das sie gebunden sind sowie den diesem benachbarten C-Atomen des Grundkörpers einen ankondensierten 6-Ring bilden,
$R^{10}$ für Wasserstoff, Methyl, Ethyl, Halogen, Amino, Methylamino, Dimethylamino steht,
$R^{11}$ für Wasserstoff, Methyl oder Ethyl steht,
n für 1 oder 2 steht.

4. 1R 3S αS und 1S 3R αR Enantiomerenpaar von trans-Z-Flumethrin mit einem Gehalt größer 80 %.

**Revendications**

1. Procédé pour la séparation de formes diastéréomères l'une par rapport à l'autre d'esters d'acides cyclopropane-carboxyliques de formule (I)

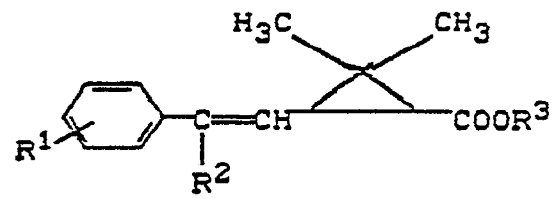

(I)

dans laquelle
$R^1$ représente l'hydrogène ou un halogène
$R^2$ représente l'hydrogène ou un halogène
$R^3$ représente un reste de formule a)

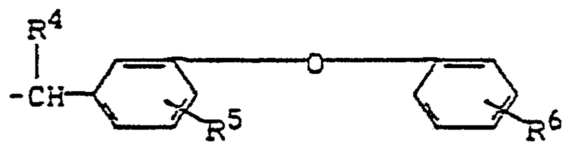

(a)

dans laquelle
$R^4$ représente l'hydrogène, CN, $-C{\equiv}CH$ ou $CH_3$,
$R^5$ représente l'hydrogène ou un halogène,
$R^6$ représente l'hydrogène ou un halogène, caractérisé en ce que, dans une première étape on fait réagir des composés de formule (I), en chauffant en présence d'un alcanol en $C_{1-4}$, avec des composés de formule (II)

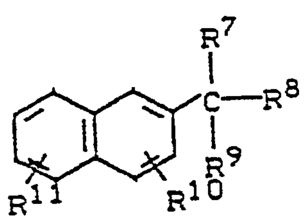

(II)

dans laquelle
$R^7$, $R^8$, $R^9$ représentent l'hydrogène ou un groupe méthyle ou forment, ensemble avec l'atome de carbone auquel ils sont liés ainsi qu'avec les atomes de carbone du corps de base qui leur sont voisins, un cycle à six chaînons condensé,

$R^{10}$ représente l'hydrogène, un groupe méthyle, éthyle, un halogène, un groupe amino, méthylamino, diméthylamino,
$R^{11}$ représente l'hydrogène, un groupe méthyle ou éthyle, de manière à obtenir un complexe dans un rapport I:II, de 1:1 ou 2:1, que, dans une deuxième étape, on cristallise et on sépare les complexes plus difficilement solubles d'une partie des diastéréomères, et que, éventuellement, dans une troisième étape, on procède à une épimérisation des complexes séparés en présence d'un solvant et en présence d'une base inorganique ou organique et on les ramène à la première étape.

2. Procédé pour purifier les esters d'acides cyclopropane-carboxyliques de formule (I) et les acides dont ils dérivent

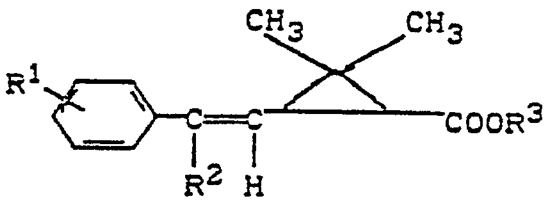

(I)

dans laquelle
$R^1$ représente l'hydrogène ou un halogène
$R^2$ représente l'hydrogène ou un halogène
$R^3$ représente l'hydrogène
ou
$R^3$ représente un reste de formule a)

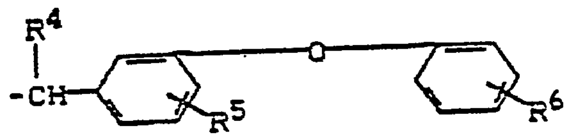

(a)

dans laquelle
$R^4$ représente l'hydrogène, CN, $-C\equiv CH$ ou $CH_3$
$R^5$ représente l'hydrogène ou un halogène,
$R^6$ représente l'hydrogène ou un halogène, des impuretés chimiques, caractérisé en ce que l'on fait réagir les composés de formule (I) en présence d'alcanols en $C_{1-4}$ et en présence de bases inorganiques ou organiques avec des composés de formule (II)

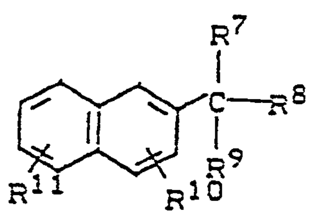

(II)

dans laquelle
$R^7$, $R^8$, $R^9$ représentent l'hydrogène ou un groupe méthyle ou forment, ensemble avec l'atome de carbone auquel ils sont liés ainsi qu'avec les atomes de carbone du corps de base qui leur sont voisins, un cycle à six chaînons condensé,
$R^{10}$ représente l'hydrogène, un groupe méthyle, éthyle, un halogène, un groupe amino, méthylamino, diméthylamino,
$R^{11}$ représente l'hydrogène, un groupe méthyle ou éthyle, dans un rapport d'environ 1:1 ou 2:1 et qu'on sépare les complexes difficilement solubles qui se forment.

3. Complexes de formule (III)

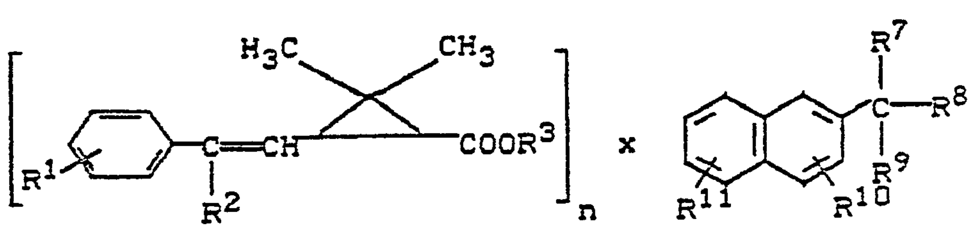

dans laquelle

$R^1$ représente l'hydrogène ou un halogène
$R^2$ représente l'hydrogène ou un halogène
$R^3$ représente l'hydrogène
ou
$R^3$ représente un reste de formule a)

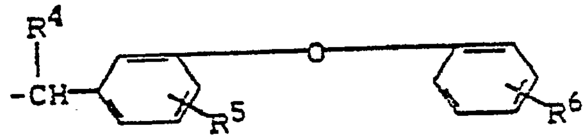

(a)

dans laquelle
$R^4$ représente l'hydrogène, CN, $-C{\equiv}CH$ ou CH3,
$R^5$ représente l'hydrogène ou un halogène,
$R^6$ représente l'hydrogène ou un halogène,
$R^7$, $R^8$, $R^9$ représentent l'hydrogène ou un groupe méthyle ou forment, ensemble avec l'atome de carbone auquel ils sont liés ainsi qu'avec les atomes de carbone du corps de base qui leur sont voisins, un cycle à six chaînons condensé,
$R^{10}$ représente l'hydrogène, un groupe méthyle, éthyle, un halogène, un groupe amino, méthylamino, diméthylamino,
$R^{11}$ représente l'hydrogène, un groupe méthyle ou éthyle,
n représente 1 ou 2.

4. Couple d'énantiomères 1R 3S αS et 1S 3R αR de trans-Z-fluméthrine avec une teneur supérieure à 80%.

## Claims

1. Process for the separation of mutually diastereomeric forms of the cyclopropanecarboxylic acid esters of the formula (I)

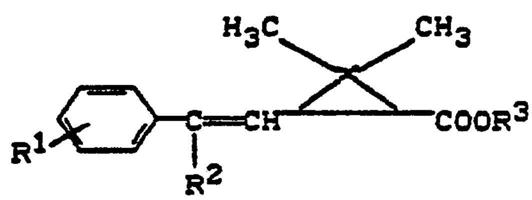

(I)

in which
$R^1$ represents hydrogen or halogen,
$R^2$ represents hydrogen or halogen, and
$R^3$ represents a radical of the formula a)

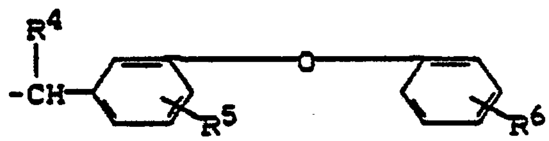

(a)

where
$R^4$ represents hydrogen, CN, $-C{\equiv}CH$ or $CN_3$,
$R^5$ represents hydrogen or halogen, and
$R^6$ represents hydrogen or halogen, which is characterized in that compounds of the formula (I) are reacted in a first stage, with heating and in the presence of a $C_{1-4}$-alkanol, with compounds of the formula (II)

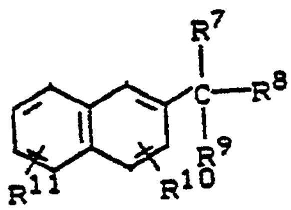

(II)

in which

$R^7$, $R^8$ and $R^9$ represent hydrogen or methyl, or, together with the C atom to which they are bound and with the C atoms, of the basic structure, neighbouring the latter, form a fused 6-membered ring,
$R^{10}$ represents hydrogen, methyl, ethyl, halogen, amino, methylamino or dimethylamino, and
$R^{11}$ represents hydrogen, methyl or ethyl, and in the I:II ratio of 1:1 or 2:1 to form a complex, and, in a second stage, the sparingly soluble complexes of one part of the diastereomers are allowed to crystallize out and are separated off, and, if appropriate, the separated-off complexes are epimerized in a third stage in the presence of a solvent and in the presence of an inorganic or organic base, and are recycled to the first stage of the process.

2. Process for the purification, from chemical impurities, of cyclopropanecarboxylic acid esters of the formula (I) and the acids on which they are based

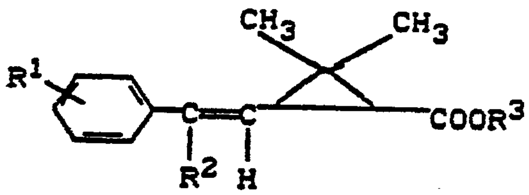

(I)

in which
$R^1$ represents hydrogen or halogen,
$R^2$ represents hydrogen or halogen, and
$R^3$ represents hydrogen, or
$R^3$ represents a radical of the formula a)

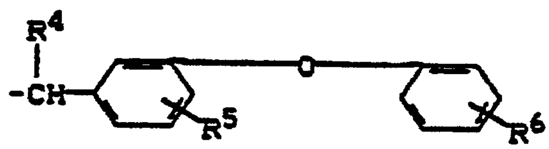

(a)

where
$R^4$ represents hydrogen, CN, $-C\equiv CH$ or $CH_3$,
$R^5$ represents hydrogen or halogen, and
$R^6$ represents hydrogen or halogen, which is characterized in that the compounds of the formula (I) are reacted, in the presence of $C_{1-4}$-alkanols and in the presence of inorganic or organic bases, with compounds of the formula (II)

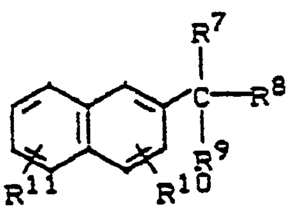

(II)

in which
$R^7$, $R^8$ and $R^9$ represent hydrogen or methyl, or, together with the C atom to which they are bound and with the C atoms, of the basic structure, neighbouring the latter, form a fused 6-membered ring,
$R^{10}$ represents hydrogen, methyl, ethyl, halogen, amino, methylamino or dimethylamino, and $R^{11}$ represents hydrogen, methyl or ethyl.

in the approximate ratio of 1:1 or 2:1 and the sparingly soluble complexes produced during this are separated off.

3. Complexes of the formula (III)

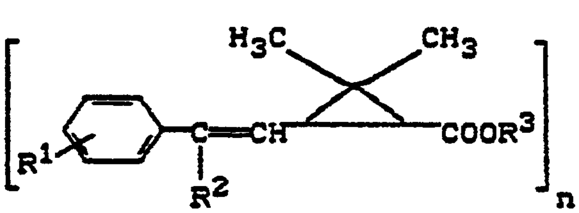

x

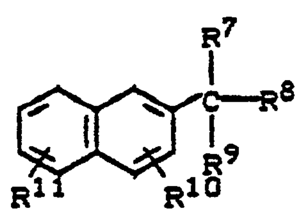

(I)

in which
$R^1$ represents hydrogen or halogen,
$R^2$ represents hydrogen or halogen, and

$R^3$ represents hydrogen, or
$R^3$ represents a radical of the formula a)

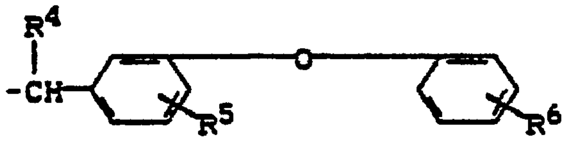

(a)

where
$R^4$ represents hydrogen, CN, $-C\equiv CH$ or $CH_3$,
$R^5$ represents hydrogen or halogen, and
$R^6$ represents hydrogen or halogen,
$R^7$, $R^8$ and $R^9$ represent hydrogen or methyl, or, together with the C atom to which they are bound and with the C atoms, of the basic structure, neighbouring the latter, form a fused 6-membered ring,
$R^{10}$ represents hydrogen, methyl, ethyl, halogen, amino, methylamino or dimethylamino, $R^{11}$ represents hydrogen, methyl or ethyl and n represents 1 or 2.

4. 1R 3S αS and 1S 3r αR pair of enantiomers of trans-Z-flumethrin having a content of greater than 80%.